(19) Europäisches Patentamt

European Patent Office

Office européen des brevets

(11) Publication number: **0 171 218 B1**

(12) **EUROPEAN PATENT SPECIFICATION**

(45) Date of publication of patent specification: **13.10.93**  (51) Int. Cl.⁵: **C12N 9/34**, C12P 19/20

(21) Application number: **85305167.0**

(22) Date of filing: **19.07.85**

(54) Enzymatic hydrolysis of granular starch directly to glucose.

(30) Priority: **06.08.84 US 637930**

(43) Date of publication of application:
**12.02.86 Bulletin 86/07**

(45) Publication of the grant of the patent:
**13.10.93 Bulletin 93/41**

(84) Designated Contracting States:
**AT BE CH DE FR GB IT LI LU NL SE**

(56) References cited:
**GB-A- 2 025 978**
**GB-A- 2 075 028**
**US-A- 3 661 716**

**CARBOHYDRATE RESEARCH, vol. 61, 1978, pages 301-308, Elsevier ScientificPublishing Company, Amsterdam, NL; P.M. TAYLOR et al.: "Some properties of aglucoamylase produced by the thermophilic fungus Humicola lanuginosa"**

**CHEMICAL ABSTRACTS, vol. 96, 1982, page 387, abstract no. 100626j, Columbus,Ohio, US; S. HAYASHIDA et al.: "Production of thermostable cellulases and rawstarch-digesting amylases by fungi", & ADV. BIOTECHNOL., [PROC. INT. FERMENTSYMP.],**

6th 1980 (Pub. 1981). 3, 271-6

(73) Proprietor: **GENENCOR, INC.**
**180 Kimball Way**
**South San Francisco California 94080(US)**

(72) Inventor: **Jackson, Leroy Eugene**
**1114 North 200 East**
**Orem Utah 84057(US)**
Inventor: **Seidman, Martin**
**346 West Macon**
**Decatur Illinois 62522(US)**

(74) Representative: **Blake, John Henry Francis et al**
**BROOKES AND MARTIN**
**High Holborn House**
**52/54 High Holborn**
**London WC1V 6SE (GB)**

## Description

Starch is a high molecular-weight polymer consisting of glucose condensed in anhydroglucose units. Complete hydrolysis of the starch polymer should yield dextrose (glucose), and starch is used to produce dextrose either as an end in itself or as a raw material for the manufacture of fructose and other products.

Starch occurs in nature in many plants in the form of discrete granules which are essentially insoluble in water at ambient temperature. Partly because of insolubility, commercial conversion of "raw" starch (starch in its original granule form) to a solution of dextrose, or to glucose syrup is a process with high energy consumption.

In the first step of commercial processes now used, an aqueous slurry of unpasted starch is heated to a temperature above its gelatinization temperature with an acid or thermally stable enzyme preparation. The purpose is to disrupt the granule structure of the starch (i.e., gelatinize it), to hydrate the starch and to hydrolyze it partially and thereby reduce its viscosity. This is necessary for operation at reasonable concentrations chosen to balance viscosity, cost of removing water and formation of certain undesirable by-products. Despite many proposals for operation in other ways, this initial step of partial hydrolysis, called "thinning," has been retained despite its cost and disadvantages.

After thinning, the starch is converted to dextrose by hydrolysis in the presence of the saccharifying enzyme glucoamylase (EC 3. 2. 1. 3). Thinning the starch with acid, commonly at a temperature of the order of 120°C, yields starch fragments and repolymerization products that are resistant to hydrolysis by glucoamylase and reduce yield. These by-products cause difficulty in filtration, and they interfere in the crystallization of dextrose and in the conversion of dextrose to high-fructose syrups especially when chromatographic enrichment is used. Color bodies, hydroxymethyl furfural and other degradation products also are produced, and these have to be removed by refining.

Enzyme thinning (with alpha amylase, EC 3. 2. 1. 1) is carried out normally at temperatures between 85°C and 110°C and higher pH than used in a saccharification reaction, but a heating step above 120°C is usually included to assure complete dispersion of the starch and improve the filtration that follows. Enzyme thinning reduces, but does not eliminate, formation of the same types of undesirable by-products. Cooling is required after thinning in all methods because glucoamylase has a relatively lower temperature optimum. Some of these problems are described in Walon U. S. Patent 4, 235, 965 issued November 25, 1980.

Another disadvantage of thinning processes is the necessity of adjusting the pH or acidity. The widely used glucoamylase preparations have optimum pHs below 5.0, and most commonly 4.0 to 4.5. When acid is used for thinning the starch, the pH must be raised to this level with alkali. Since alpha amylase has a pH optimum above 5.0, the pH has to be reduced to the optimum for glucoamylase. In either case, the change in pH introduces ions that must later be removed.

The present invention makes possible the direct conversion of granular starch, i.e. raw starch in aqueous slurry (suspension), to dextrose (glucose) in solution. This is achieved through the use of a new enzyme preparation having a unique combination of properties. This enzyme preparation is made by a number of thermophilic and mesophilic fungi which have been discovered and identified. The enzyme preparation is noteworthy for its ability to convert insoluble starch granules directly to dextrose at temperatures below the swelling temperature of starch at starch concentrations high enough for commercial glucose production, and without an intermediate thinning or solubilizing step and without pasting or swelling the starch.

## SUMMARY OF THE INVENTION

In one aspect, the present invention provides an enzyme preparation obtainable from a substantially pure culture of a fungal organism which is a strain of Humicola grisea var. thermoidae, which enzyme preparation catalyzes the hydrolysis of granular starch substantially directly to glucose, said enzyme preparation being characterized in that

(a) it catalyzes the hydrolysis of granular starch suspended in water at a concentration of 15% by weight starch solids substantially completely to soluble glucose syrup solids containing at least 97% by weight glucose, dry substance basis, when the hydrolysis is carried out at a pH of 5.0 to 7.0 and a temperature of 55°C and without added alpha amylase or added debranching enzyme of the pullulanase, isoamylase or beta amylase type,

(b) it is separable by carboxymethyl cellulose into a first non-adsorbing fraction and a second adsorbing proteinaceous fraction, said first non-adsorbing fraction containing a glucoamylase-potentiating activity and said second adsorbing fraction having a glucoamylase enzyme activity (EC 3.2.1.3) that has an isoelectric point of 8.0 or higher.

2

In another aspect, the present invention provides a fungal organism which is a strain of Humicola grisea var. thermoidae deposited under reference NRRL 15219, NRRL 15220, NRRL 15221, NRRL 15222, NRRL 15223, NRRL 15224 and NRRL 15225, or is a genetically altered strain artificially derived from any of said organisms and having the ability when cultured by the Standard Culture Procedure as defined herein to produce an enzyme preparation as characterized in Claim 1 having an RSH (Raw Starch Hydrolysis) activity of 120 units/ml or more as assayed by the Standard Assay Procedure Number 1 as defined herein.

In a further aspect, the present invention provides a glucoamylase enzyme fraction obtainable from a substantially pure culture of a fungal organism which is a strain of Humicola grisea var. thermoidae, characterized as having an isoelectric point of about 8.0 or higher than 8.0 capable of being adsorbed on carboxymethyl cellulose and exhibiting maximum activity at a pH of between 5.0 and 7.0.

In a yet further aspect, the present invention provides a potentiating enzyme fraction which along with a glucoamylase enzyme obtainable from a substantially pure culture of a fungal organism which is a strain of Humicola grisea var. thermoidae, catalyzes the hydrolysis of granular starch suspended in water, at a concentration of 15% by weight starch solids, substantially completely to soluble glucose syrup solids containing at least 97% by weight glucose, dry substance basis, when the hydrolysis is carried out at a pH of 5.0 to 7.0 and at a temperature of 55°C and without added alpha-amylase or added debranching enzyme of the pullulanase, isoamylase or beta-amylase type.

The invention is directed to solubilizing and hydrolyzing raw starch to produce a carbohydrate composition that is predominantly glucose. The process includes the production of a fungi fermentation broth containing a mixture of enzymes that is characterized by including a glucoamylase enzyme (EC 3.2.1.3) having an isoelectric point of about pH 8.0 or higher and a proteinaceous material having glucoamylase-potentiating activity which, in cooperation with the glucoamylase, catalyzes the dissolution of granular starch. The enzyme mixture is further characterized in that the glucoamylase adheres to carboxymethyl cellulose gel, whereas the potentiating material (hereinafter "potentiating factor") is not adsorbed by carboxymethyl cellulose cation exchanger. The enzyme preparation has the ability, for example, to hydrolyze granular starch in a 15% starch solids suspension in water to a solution of saccharides of at least 97% dextrose, dry solids basis, with essentially no starch residue and less than 1% trisaccharide and higher anhydroglucose polymers in the absence of debranching enzyme or added alpha amylase when the hydrolysis is carried out at a pH of about 5.0 to 7.0, the optimum pH.

The enzyme mixture is expressed as an extracellular enzyme preparation by certain fungi, particularly fungi of the genus Humicola. A preferred species of the genus Humicola from which the enzyme mixture may be isolated, is Humicola grisea var. thermoidea. Mutants of this species have been induced which exhibit raw starch hydrolyzing activity well in excess of that produced by the wild type.

In particular, mutant pure cultures of strains of H. grisea var. thermoidea NRRL 15219; NRRL 15220; NRRL 15221; NRRL 15222; NRRL 15223; NRRL 15224 and NRRL 15225 have been induced. Of these, a highly preferred species is NRRL 15219. These produce enzyme preparations that are immunologically the same, and the isolated glucoamylase fraction and potentiating factor fraction also match the corresponding fractions immunologically.

An effective amount of the fermentation broth is used to hydrolyze granular starch in aqueous suspension, e.g., with at least about 15% starch solids, without added alpha amylase or debranching enzyme to produce syrup containing over 95% dextrose on a dry substance basis.

The glucoamylase enzyme found in the enzyme preparation of this invention is also useful in the hydrolysis of various intermediate products derived from granular starch. Glucoamylase is commonly used for the hydrolysis of thinned starch hydrolyzates to yield glucose. The glucoamylase of this invention will hydrolyze thinned starch hydrolyzates to glucose in a manner similar to known glucoamylases. However, the present glucoamylase has an optimum pH in the range of 5.0 to 7.0 as compared to an optimum pH of less than 5.0 and most commonly 4.0 to 4.5 for widely used glucoamylase preparations. The higher optimum pH of the glucoamylase of this enzyme preparation reduces the pH adjustments necessary in the thinned starch to glucose conversion process. The use of the glucoamylase fraction of the enzyme preparation reduces the need for ion removal after the hydrolysis of thinned starch hydrolyzate to glucose is completed.

DRAWING

The Figure is a graph showing the changes in dextrose content and starch dissolved during the course of comparable hydrolyses using the enzyme preparation of this invention and using an alpha amylase/glucoamylase combination.

3

## DETAILED DESCRIPTION OF THE PREFERRED EMBODIMENTS

In all known prior processes, the hydrolysis of starch proceeds through the formation of soluble intermediate polymers of glucose, dextrins or oligosaccharides. Surprisingly, the enzyme preparation of this invention has the ability to hydrolyze normally insoluble granular starch directly to glucose in solution, without first producing soluble intermediate or dextrinlike starch hydrolysis products in solution. If they form, intermediate hydrolysis products appear to remain in the granules, and the dextrose content of the solution solids is substantially 100% at first, decreasing somewhat with time but still remaining over 90% even at very high initial starch concentrations. At 16% starch solids initial concentration, the glucose concentration remains at 99% or more of the saccharides in solution. Typically, even at 25% solids, glucose content is over 97% after 24 hours and in 72 hours decreases toward 95% after hydrolysis of over 80% of the starch with a gradual increase in disaccharides (D.P.2 anhydroglucose polymers). This is entirely unlike prior processes in which there is a continuing and gradual increase in the dextrose content of the solids in solution, with early formation of soluble intermediate polysaccharide or oligosaccharide chains.

The enzyme preparation of this invention was first isolated from the strain of H. grisea var. thermoidea ATCC 16453 (1982 catalog, p. 389). This organism is described in Cooney and Emerson, "Thermophilic Fungi" (1964, Freeman, San Francisco). The recommended culture medium (ATCC catalog, supra) was Emerson YpSs agar which contains soluble starch (Cooney and Emerson, page 13). The enzyme of this invention was first obtained by growing this culture in a liquid medium of corn steep liquor, mineral salts, 0.1% pasted starch and 1-2% granular starch that had been sterilized by overnight storage under diethyl ether. The pasted starch is substantially less than optimum for growth of the organism, but was sufficient to initiate growth. It was noticed that the granular starch was degraded.

Enzyme made in this way was first separated by precipitation using two parts of isopropanol added to one part of growth medium. This isolated enzyme rapidly degraded granular starch to produce predominantly glucose both at pH 4.5 and, surprisingly, at pH 6.0, a pH at which glucoamylase is generally believed to be inactive. The enzyme produced in a medium containing granular starch was compared with enzyme made in a medium containing only pasted starch. The latter converted granular starch to dextrose at about 92% and the former to 96% .

## DESCRIPTION OF ART

The desirability of converting starch directly from its granular form to dextrose (glucose) has been the goal of much research, especially for use with starch from corn (maize), the world's principal source of glucose syrups and crystalline dextrose. Recent patents are Leach et al U . S. Patents 3,922,196 and 3,922,197 issued November 25, 1975 and Marshall U. S. Patent 4,234,686 issued November 18, 1980 and 4,318,989 issued March 9, 1982. Leach et al used bacterial alpha amylase with or without glucoamylase to obtain a soluble partial hydrolysate that could then be converted to dextrose with glucoamylase. Marshall used an enzyme having pullulanase activity and other amylases with debranching or dextrinizing activity.

On the other hand, there have been many more or less academic studies at dilute concentrations. Reference may be made to studies such as those of: 1) Ueda and others: "Raw Starch Digestion by Mold Glucoamylases and Debranching Enzymes" in Mechanisms of Saccharide Polymerization and Depolymerization edited by J. J. Marshall (1980) pp. 55-72; Trends in Biochemical Sciences (TIBS) (March, 1981) pp. 89-90; Staerke 33 (1981) 313-316; ibid. 32 (1980) pp. 122-125; ibid. 31 (1979) pp. 307-314; ibid. 28 (1976) pp. 20-22; ibid. 27 (1975) pp. 123-127; 2) Hayashida and others: Agric. & Biol. Chem. 46 (1982) pp. 83-89; ibid. 39 (1975) 2093-2099; 3) Fuwa et al. : "Degradation of Various Starch Granules by Amylases" in Marshall, supra, pp. 73-100; and by many others. These show some susceptibility of granular starch to hydrolysis by many glucoamylase preparations, but with high conversions occurring only at low starch concentration.

The enzymes that have been used to enhance conversion of granular starch by glucoamylase are characterized by the formation of short chain-length oligosaccharides, e.g., maltose, maltotriose and dextrins, during hydrolysis. A recent publication (Wankhede et al, Staerke, 34 (1982) pp. 309-312) deals with the "synergistic" effects of alpha amylase or pullulanase (EC 3.2.1.41) on glucoamylase during the digestion of raw starch, but even under these conditions in very dilute suspensions, less than 70% of the starch was converted in 60 hours.

The 1981 Ueda review article in TIBS, supra, is a review of glucoamylase preparations for the digestion of raw starch in the presence of yeast to make alcohol. Reference is made to glucoamylase preparations obtained from molds that are said to have raw-starch digestion properties at least at dilute concentrations. Reference is made to Humicola lanuginosa, but no significant difference in the activity of its glucoamylase was given. This review also concludes that glucose and maltose inhibit the digestion of raw starch and the adsorption of enzyme to it. The glucoamylase produced by Humicola lanuginosa was the subject of a study by Taylor et al (Carbohydrate Research, 61 (1978) pp. 301-308), and the properties of other fungi of the Humicola group, namely H. grisea var. thermoidea and H. insolens are given by Ellis (Trans. Br. Mycol. Soc. 78 (1982) pp. 129-139). A review of some properties of thermophilic fungi, with emphasis on pH and temperature optima is given by Rosenberg in Can. J. Microbiol. 21 (1975) pp. 1535-1540. This article contains data on members of the Humicola group; it is noted that H. lanuginosa (ATCC 16455) is the same as Thermomyces lanuginosa. These articles do not disclose the use of glucoamylase to convert granular starch to dextrose.

In U.S. Patent 4,247,637 issued January 27, 1981 to Walon, there is a comparison between the enzyme produced by Humicola lanuginosa (Thermomyces lanuginosa) and the enzyme of the invention claimed in the patent, produced by a Talaromyces strain. This patent discloses that the glucoamylase of the T. lanuginosa organism was the most heat-stable glucoamylase preparation at the time, but was rapidly inactivated at pH 6.0 and 70°C. The optimum pH and temperature of the enzyme claimed in this patent was said to be 4.0 and 75°C, respectively, and the values for the Humicola species enzyme were 6.5 and 65°C, respectively.

In U. S. Patent 4,435,307 issued March 6, 1984 to Barbesgaard et al, there is a disclosure of a cellulase enzyme preparation from Humicola insolens, DSM 1800. The taxonomic distinction of H. insolens and H. grisea var. thermoidea is said to be "dubious", evidently based on the publication cited above by Ellis who based his statement on electron microscopy. Cooney and Emerson in Thermophilic Fungi distinguish the two by the color and by the absence of phialospores from H. insolens. Emerson's classification is apparently based upon the observation that chlamydospores in H. grisea var. thermoidea are borne singly on short lateral aleuriophores and are rarely found as intercalary spores. Examination of the H. grisea var. thermoidea fungi of this invention regularly showed both terminal aleuriospores and intercalary chlamydospores, confirming Emerson's opinion. The term "intercalary" is applied to a spore that develops within a filament or hypha of a mold.

In accordance with the present invention, it is discovered that certain fungi, particularly members of the genus Humicola, secrete an enzymatic mixture of proteins (enzyme preparation) in the fermentation broth that hydrolyze raw or granular starch, including straight- and branched-chained starches and hydrolyze the starch substantially entirely to glucose. The enzyme mixture is characterized by including a glucoamylase enzyme (EC 3.2.1.3) having an isoelectric point higher than pH 8.0 and a proteinaceous material having glucoamylase-potentiating activity which, in cooperation with the glucoamylase, catalyzes the dissolution of granular starch. The enzyme mixture is further characterized in that the glucoamylase adheres to carboxymethyl cellulose cation exchanger, whereas the potentiating material (hereinafter "potentiating factor") is not adsorbed by carboxymethyl cellulose gel. The enzyme mixture has the ability, for example, to hydrolyze granular starch in a 15% starch solids suspension in water to a solution of saccharides of at least 97% glucose, dry solids basis, with essentially no starch residue and less than 1% trisaccharide and higher anhydroglucose polymers in the absence of debranching enzyme or added alpha amylase when the hydrolysis is carried out at a pH of about 5.0 to 6.5, the optimum pH.

Although the raw starch hydrolyzing enzymatic activity was first discovered in a wild type of Humicola species, various mutants have been artificially induced which yield elevated amounts of the raw starch hydrolyzing (RSH) enzymatic activity relative to known wild types. Mutant strains which when germinated and used in fermentation, as hereinafter described, produce broths having RSH enzymatic activity of 120 units per ml and upwards, when assayed as hereinafter described. Increased enzymatic activity of broths produced by mutant strains may arise from increased enzyme production by individual organisms and/or from increased growth of the species under the germination and fermentation conditions. In most of the mutants thus far developed, it appears that the increase in secreted enzymes results primarily from increased enzyme production by the individual organisms rather than from more rapid growth of organisms. This is considered fortuitous because a higher enzyme production is obtained using a similar amount of carbohydrate feedstock, e.g., starch, during fermentation to produce the broth.

Mutant strains which produce broth with elevated RSH activity represent increased potential for full commercialization of the process of the present invention. Although wild type fungi produce broth having RSH activity, the amount of production is generally less than that desirable for large-scale starch hydrolysis. Preferably, a fungi used for producing RSH enzymatic mixture produces a broth having an activity of 250 units per ml or above.

Mutations may be induced by a variety of methods known in the art, including exposure to mutagenic chemicals and irradiation of various types. Several useful Humicola mutants have been developed in this manner. Mutant species are selected for their ability to produce high titers of RSH activity as well as for

EP 0 171 218 B1

other desirable characteristics. Furthermore, apparently useful mutants are passaged at least three times to assure the stability of their acquired genetic characteristics.

In addition to producing high titers of enzyme, it is desirable that a species grow at relatively elevated temperature, and herein mesophilic fungi, which grow at temperatures of 37°C to 40°C, and thermophilic fungi, which grow at temperatures of 40°C to 50°C, are preferred species for producing the RSH enzymatic mixture. An important advantage of thermotolerant fungi is that when grown at the relatively elevated temperatures, those contaminating fungi and other contaminating organisms which cannot survive at such temperatures are eliminated. A further advantage of growing at elevated temperatures is that the organisms tend to grow faster and therefore produce higher titers of RSH enzyme preparation.

The enzyme mixture exhibiting RSH activity as described above is used in a process for producing dextrose directly from raw starch. The process includes germination of fungi spores, a fermentation using the germinated fungi, separation of the fermentation broth from the cultured organisms and hydrolysis of raw starch using the separated broth. Although dextrose is produced when raw starch is fermented with the fungi that produce the RSH enzyme mixture, yields of dextrose are relatively low, presumably because the dextrose that is produced is consumed by the culture. Thus, separation of the enzymatically active broth for use in hydrolyzing the raw starch appears to be necessary for good dextrose yields. Furthermore, even though the fungi are mesophilic or thermophilic, growing at relatively elevated temperatures, the enzymatic mixture exhibits maximum catalytic activity at temperatures above temperatures conducive to growth of the organism, e.g., about 55°C. By separating the broth-producing fermentation step from the enzymatic hydrolysis step, the temperature may be optimized for the fermentation step as well as for the enzymatic hydrolysis step. In addition, the separation procedure allows the removal of organisms, spores and other contaminants that may affect the color and quality of the resulting dextrose syrup.

The procedure begins with germination of fungi from spores to produce an inoculum for a subsequent fermentation. A sterile substrate is provided containing sources of carbohydrate, such as thinned starch, the carbohydrate comprising between about 10 and about 40% by weight of the substrate. The pH of the medium is adjusted to between about 4.5 and about 7.0 and preferably between about 5.0 to about 6.5. The substrate is inoculated with enough inoculum to provide between 50,000 and 500,000 spores per ml of medium. The spores are germinated for several days, typically between about 3 and about 7 days and at a temperature conducive to germination of the individual species or variety. The germinated fungi may be stored refrigerated until use.

The germinated spores are used as inoculum for a fermentation medium that includes a carbohydrate substrate. Typically a sterile liquid fermentation medium is provided into which the germinated fungi is inoculated. The liquid medium provides a carbohydrate substrate, such as a low concentration of pasted starch as well as a source of other nutrients including proteins and vitamins. The liquid medium also provides nitrogen. Corn steep liquor is a preferred nutrient source being relatively inexpensive. The liquid medium is also buffered to a pH of between about 4.5 and about 7.0 and preferably between about 5.0 and 6.5 with inorganic salts conducive to fungal growth. In addition, anti-bacterial agents, such as penicillin G and oxytetracycline may be added. The total fermentation medium also preferably contains at least some raw starch. The raw starch is preferably added to the liquid medium after the liquid medium has been inoculated.

The raw starch that is added to the fermentation medium appears to induce the production of RSH enzyme mixture. That is, the same species or variety of fungus produces a higher titer of RSH activity if at least some raw starch is present in the fermentation medium than if none is present.

The fermentation is carried out at a temperature conducive to growth of the fungus and preferably the temperature is optimized to the particular species or variety. Preferably, the fermentation mixture is continuously agitated and aerated. Fermentation is carried out for a time period sufficient for the culture to secrete a substantial titer of enzyme mixture into the broth, typically between about 24 and about 84 hours.

After fermentation, the broth is separated from the solid materials, such as the mycelia. Separation may be effected by filtration, centrifugation, or other techniques known in the art to be suitable for separating solids from liquid. The separated enzyme broth is suitable for hydrolyzing raw starch without further purification. It is found that enzyme production tends to reach a maximum if the fermentation proceeds for about 48 hours. If the fermentation proceeds longer, the enzyme production levels off and may even decline.

The separated fermentation broth is usable without further purification to hydrolyze raw starch, and it is generally most economical to use the crude broth without purification. The raw starch is provided in an aqueous slurry, and for efficient production of glucose, the raw starch content is at least about 15% by weight dry substance starch and preferably at least 25% dry substance starch and may be up to about 60% dry substance starch. The optimal pH for hydrolysis is around 6.0 but hydrolysis may be carried out at any

6

pH in the 5.0 to 7.0 range. The optimal temperature for hydrolysis with the RSH enzymatic mixture is about 55°C, but enzymatic activity is lost at temperature above about 60°C. At lower temperatures, down to 0°C, hydrolysis occurs but at increasingly lower rates. However, it is generally considered that hydrolysis should be performed at 40°C or above for efficient glucose production. The broth is added to the slurry to provide between about 10 and about 300 units of enzyme activity per gram of starch and preferably between about 25 and about 100 units of enzyme activity per gram of starch. A low titer of enzyme naturally produces glucose relatively slowly. On the other hand, the amount of glucose produced per unit of enzyme does not appear to be linear, and high titers of enzyme produce proportionately less dextrose per unit than does a moderate titer. Accordingly, the titer of enzyme is selected which produces glucose at a fairly rapid rate and yet at a generally optimal rate per unit of enzyme.

ENZYME CHARACTERISTICS

The enzyme preparation of this invention catalyzes the hydrolysis of granular starch whether in isolated form, in the form of dry milled products or in the matrix of vegetable tissue. It has been used to remove residual starch from corn bran separated in the corn wet milling process. The enzyme is useful in the hydrolysis of cereal starch, such as corn (maize), wheat, rice and of root and tuber starch, such as white and sweet potato and tapioca, as well as straight- and branched-chain starch molecules, i.e., amylose and amylopectin, whether as isolated fractions or as waxy starch or high-amylose corn starch. Because of its glucoamylase activity, the enzyme of this invention can also be used in the hydrolysis of pasted starch, soluble starch, low D.E. starch hydrolyzates (e.g., D.E. of 2 to 20) and other starch-like glucose polymers.

Recovery and Fractionation of Enzyme

A distinctive aspect of this invention is the presence of the potentiating factor which in addition to glucoamylase appears to make possible the hydrolysis of granular starch. The potentiating factor, as fractionated from purified enzyme preparation, is a mixture containing proteins of undetermined structure with components which have been identified by their enzymatic activity.

Enzyme preparation of this invention is recovered from the fermentation medium by filtering or centrifuging to remove mycelia, debris and other residue. If the solution is to be concentrated, the pH of the filtrate is adjusted to 6, and it is concentrated by vacuum evaporation or ultrafiltration. Instead of concentrating the enzyme preparation, it can be precipitated at 4°C with acetone (volume for volume) or with diammonium sulfate (50% of saturation) and recovered by centrifugation. Acetone precipitation is preferred. To a person skilled in the art, it will be obvious there are other methods to recover the enzyme preparation.

To purify the raw-starch hydrolyzing ("RSH") activity for study, acetone-precipitated enzyme is treated with diethylaminoethyl ("DEAE") cellulose. Unlike substantially all other glucoamylase preparations, the enzyme preparation of this invention is not adsorbed by DEAE cellulose (Whatman pre-swollen DEAE cellulose DE-52 was used); instead, inactive protein is removed, thereby concentrating the desired activity (glucoamylase and potentiating factor) in the effluent. The procedure is equally effective when carried out in column or in batch at a pH of about 5.0 to 7.0 (preferably 6.5 to 7.0) with a 2- to 4. 5-fold increase in specific activity (units per gram or units per milliliter). The increase is at least partly attributable to the removal of inactive protein. The product is referred to as "purified" enzyme preparation.

In order to separate the fraction containing the potentiating factor, the purified enzyme preparation was subjected to cation exchange chromatography using carboxymethyl cellulose as adsorbent. Glucoamylase enzyme is strongly adsorbed as a single protein band that can be eluted with dilute NaCl, and the fraction containing potentiating factor is not adsorbed.

The "potentiating factor" is the fraction of the enzyme preparation that is not adsorbed from purified enzyme preparation by column chromatography using carboxymethyl cellulose ion exchange material under these conditions:

Column: 2.5 cm x 13.4 cm Whatman CM-52 carboxymethyl cellulose equilibrated with 10 mM sodium phosphate buffer, pH 6.8.

Sample: 50 ml purified enzyme preparation, 0.43 mg/ml protein, 40 units per ml or equivalent.

Wash: 110 mls 10 mM sodium phosphate, pH 6.8.

Linear Gradient: 400 ml of 10mM sodium phosphate, pH 6.8, 0 to 0.5M sodium chloride, initiated following wash.

Flow rate: 2 ml/minute.

Fraction volume: 9 ml to 12 ml.

The potentiating factor is eluted during the column washing while the glucoamylase is strongly adsorbed. Glucoamylase is eluted by the linear gradient between 50 mM and 200 mM sodium chloride. Recovery is over 80% of the glucoamylase units at a concentration of about 0.1 mg protein per ml. Each fraction can be preserved by freezing or by freeze-drying.

Separation of protein components of the potentiating fraction by the technique called "chromatofocusing" ("Chromatofocusing with Polybuffer and PBE", Pharmacia Fine Chemicals, Nov., 1980), and testing showed it to have the following activities: alpha-glucosidase, beta-glucosidase, cellulase, glucoamylase, alpha amylase and xylanase. The following were undetected: beta amylase, pullulanase, isoamylase, protease, dextranase, isomaltase, fructosyl transferase, invertase and alpha-1,6-galactosidase. A relatively low level of alpha amylase activity was also inferred from the enhancement effect of calcium ion, inhibition by EDTA, loss of activity when pH is reduced below 5 (alpha amylase denatured) and activity against amylopectin (waxy starch). However, the saccharides produced from starch by the action of potentiating factor alone are entirely different from the saccharides produced by alpha amylase: about one-third dextrose, one-third polysaccharide (D.P. 10 and above) and D.P. 3 saccharide content higher than D.P. 2 (no alpha-1,6 linkages). Alpha amylase does not produce dextrose. Further, independently measured alpha amylase activity is very low, far less than can account for the ability of the enzyme preparation of this invention to hydrolyze the starch in granule form without substantial formation of soluble short-chain polysaccharides. This is further illustrated hereafter.

That the potentiating factor is none of the common carbohydrase enzymes is shown by the data in Table 1 in which potentiating factor is compared with alpha amylase, pullulanase and isoamylase as well as with glucoamylase. The substrates used were Lintner (soluble) starch, Sigma brand potato amylopectin, potato amylose and pullulan. The enzymes were: Thermamyl 120L bacterial alpha amylase, Hayashibara isoamylase and Novo pullulanase SP 247. All hydrolyses were carried out at pH 5.5 and 50°C for 30 minutes at a substrate concentration of 1%, except amylopectin was used at 0.5% because the product supplied exhibited a relatively high reducing sugar blank. Reducing sugar was measured by the Somogyi-Nelson procedure (Starch and Its Derivatives, Radley, Editor, 4th Ed. , p. 431), and dextrose was determined on a Yellow Springs Instrument industrial analyzer. The ratio of reducing sugars to dextrose should be 1.0 for glucoamylase on starch. The blanks in the Table indicate that, when amylose or amylopectin was used, no dextrose was found and that when pullulan was used, no reducing sugar was found.

TABLE 1

| Ratio Reducing Sugars/Dextrose | | | | |
|---|---|---|---|---|
| | Soluble Starch | Amylopectin | Amylose | Pullulan |
| Potentiating fraction | 2.1 | 2.2 | 2.0 | 0.74 |
| Glucoamylase fraction | 0.94 | 1.3 | 0.77 | 0.70 |
| Alpha amylase | 100 | ----- | 18 | ---- |
| Isoamylase | 110 | ----- | ----- | 1.8 |
| Pullulanase | 6.3 | 120 | ----- | 1.8 |

Optimum pH and pH Stability

The range for the enzyme preparation of this invention in the hydrolysis of granular starch is 5.0 to 7.0 with maximum activity at pH 5.5 to 6.0. Values were determined with 0.69 and with 2.5 enzyme activity units/ml reacted with 17% (w/v) granular corn starch at 49°C for one hour in sodium acetate buffer at a pH under 6.0 and in sodium phosphate buffer at pH 6.0 and above. Undissolved starch was removed by centrifuging, and the reaction was quenched by dilution and boiling the supernatant.

The pH stability of the present enzyme preparation was measured by holding at pH levels from 2 to 8 for one hour at 25°C and for 20 minutes at 50°C. At both temperatures, over 90% of the activity was retained in the pH range 5 to 8, and the most stable range is pH 6 to 7.

The activity of the enzyme of this invention in thinned starch (D.E. 10 maltodextrin) is slightly lower than glucoamylase from A. niger, A. oryzae, or R. niveus at pH 4.5. Below pH 5.0, the potentiating factor activity is lost so that the enzyme of this invention is similar in its action on granular starch to other glucoamylase preparations.

8

Thermal Stability

The thermal stability of enzyme preparation in the absence of starch was measured between 50 and 60°C by holding samples of enzyme preparation, as is, at pH 6.7 for 20 minutes at each temperature and assayed using 10 D.E. maltodextrin. Over 90% of the activity is retained at 53°C, and over 55% was retained at 57°C compared to samples held at room temperature.

Mode of Hydrolysis

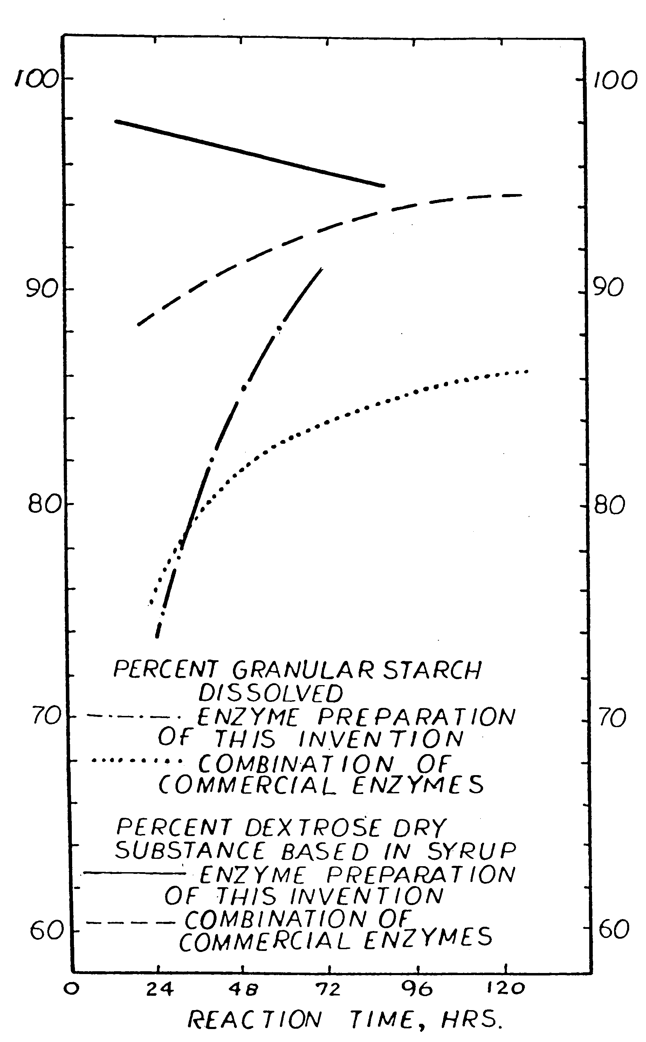

The enzyme preparation of this invention is distinguished from prior enzyme compositions used for starch hydrolysis, especially on granular starch, in the mode of hydrolysis as determined from the soluble saccharides formed. In the present invention, during the initial hours of hydrolysis, substantially the only saccharide present in solution is dextrose (glucose), i.e. , on a dry saccharide solids basis the solution is substantially 100% glucose. Following the initial stage of solution of the starch, there appears to be a gradual increase of higher saccharides, apparently formed by recombination or repolymerization of the glucose. At the end of 24 hours, the glucose content of the liquid from a 15% or more starch slurry may be reduced from 100% to 97-98%, dry substance basis, with gradual further decline to 96-97% after 48 hours and 95-96% after 72 hours. In contrast, a typical hydrolysis of granular starch with a combination of alpha amylase and glucoamylase will show a dextrose content of less than 90% after 24 hours, rising to 92-93% after 72 hours and leveling off below 95%. This is shown graphically in Figure 1 and details are given in Example 5 hereinafter.

As is well known, the hydrolysis of starch in the presence of glucoamylase, when continued for extended periods of time, produces a peak in dextrose concentration. Under commercial conditions using 10-20 units of glucoamylase per gram, the maximum dextrose content is normally reached in about 48-72 hours, and thereafter, the dextrose content decreases slowly as repolymerization occurs to produce short-chain anhydroglucose polymers that are resistant to hydrolysis. The present enzyme preparation does not have such a peak; instead, the earliest measurements of dextrose content of the starch hydrolyzate are essentially 100% dextrose and there is only a slow decline in concentration, as illustrated in Figure 1.

Thirty units of the enzyme of this invention per gram of starch resulted in complete dissolution of granular starch at 16% solids in 48 hours at pH 5.7 and 55°C. The saccharide in solution is over 95% dextrose and no starch-like substance is detectable by response to iodine as starch responds, i.e. , there is no anhydroglucose polymer that forms an iodine complex.

Isoelectric Point

The isoelectric point of the glucoamylase fraction of the enzyme of this invention is higher than that measured for most other glucoamylase preparations. The isolated glucoamylase fraction had isoelectric point above pH 8.0. Values measured for the three enzyme preparations A. niger, A. oryzae and R. niveus were 3.7, 3.4 and 7.7, respectively (literature: 3.4 to 4.0, 3.4 to 3.5 and 8.7 to 8.8, respectively). The measured isoelectric points were determined with the Isogel Agarose Isoelectric Focusing procedure ("A Step by Step Guide to Isogel Agarose Isoelectric Focusing". FMC Marine Colloids Division BioProducts, January, 1980).

Molecular Weight

By means of the SDS polyacrylamide gel electrophoretic method of Laemmli (Nature, 227 (1970) pp. 680ff. ), glucoamylase and potentiating factor fractions were resolved, and molecular weights were determined. The gels used were 7% and 10% acrylamide, each with 2.7% cross-linking. The glucoamylase enzyme molecular weight was determined as 64,300, and the molecular weights of the three major components of the potentiating factor were measured as 87,300, 72,500 and 52,200, respectively.

Sensitivity to Metal Ions

The metal ions tested were calcium, chromium, ferric, zinc, magnesium, manganese, nickel. The only observed effect on the hydrolysis of granular starch was a slight enhancement in rate by calcium ion, but this occurred only in the initial stages of hydrolysis.

Morphology

As noted above, the first sample of enzyme of this invention was isolated from Humicola grisea var. thermoidea ATCC 16453, an organism described in the literature. ATCC 16453 was progressively mutated by subjecting it and its derivative varieties to N-Methyl-N'-Nitro-N-Nitrosoguanidine or a germicidal ultraviolet light (253 nm wave length). Samples of ATCC 16453 and of each of the mutants developed were grown on YpSs agar of the following composition:

| Yeast extract (Difco) | 4.0 gm |
| $K_2HPO_4$ | 1.0 gm |
| $MgSO_4.7H_2O$ | 0.5 gm |
| Soluble starch | 15.0 gm |
| Distilled water | 1000 ml |
| Agar (Difco) | 15 gm |

The morphology of the mutants derived from this organism differs from that of ATCC 16453 in the following respects:

Turf: Whereas ATCC 16453 is dark gray with undulated surface and somewhat irregular furrows with relatively well-defined edges cut into the agar, the mutants show the following characteristics, all being more highly pigmented than ATCC 16453 (more black):

NRRL 15219 - brownish gray with smooth surface and small furrows.

NRRL 15220 - gray brown with smoother surface and less furrowed than ATCC 16453.

NRRL 15221 - brown shading to gray with slightly undulant surface.

NRRL 15222 - gray with relatively broader furrows tending to undulant surface.

NRRL 15223 - gray with brown tinge and smooth surface with slight creasing; lawn not completely confluent.

NRRL 15224 - light gray with discrete colonies and lawn not confluent.

NRRL 15225 - dark gray to black, rough, surface growth not confluent, pigment in agar.

Reverse: Whereas ATCC 16453 was dark brown to gray and peach-colored at 30°C, the mutants showed the following, all having more pigmentation (black) than ATCC 16453:

NRRL 15219, -221 and -223 - dark gray to black.

NRRL 15220 and -224 - gray to black.

NRRL 15222 - gray to black, nearly black.

NRRL 15225 - black.

Aleuriospores and chlamydospores: The mutants were indistinguishable from the original strain of ATCC 16453, and the classic description of Emerson and Cooney (Thermophilic Fungi, chapter 8) was confirmed. Comparison of strains of Humicola insolens (ATCC 16454 and ATCC 22082), grown under the same conditions, showed them to fit the descriptions in Emerson and Cooney.

Temperature Limits: Observations were made on samples grown for 5 days on agar of the composition set forth above at temperatures of 25, 30, 37, 42, 45, 50 and 55°C. Observations are displayed in the tables of growth and sporulation following. In the headings, "good growth" signifies a luxuraint lawn, "moderate growth" somewhat less and "light" is used to describe growth in which the plate is not covered completely. None of the samples were observed to grow at 55°C. The amount of sporulation increases rapidly with temperature and dramatically affects the color (pigmentation) of both the colony and the medium. Heavy sporulation produces gray to black. The words "none visible" refer to growth with little or no pigmentation, i.e., almost white, whereas the term "light" is used to refer to those plates with little pigmentation, usually appearing as tan.

## EFFECT OF TEMPERATURE (°C) ON GROWTH
### (Growth temperatures: 25, 30, 37, 42, 45, 50, 55*)

### GROWTH RATING

| Strain | None | Light | Moderate | Good | Moderate | Light |
|--------|------|-------|----------|------|----------|-------|
| ATCC | | | | | | |
| 16453 | | 25 | | 30, 37, 42, 45 | 50 | |
| NRRL | | | | | | |
| 15219 | | 25, 30 | 37 | 42, 45, 50 | | |
| 15220 | | 25, 30 | 37 | 42, 45 | 50 | |
| 15221 | 25 | 30 | 37 | 42, 45 | | 50 |
| 15222 | | 30 | | 37, 42, 45, 50 | | |
| 15223 | 25 | 30 | | 37, 42, 45, 50 | | |
| 15224 | 25 | 30*,37 | 42 | | | 45,50 |
| 15225 | 25 | 30 | 37 | 42, 45, 50 | | |

*No growth of any of the samples was observed at 55°C under these conditions.

## EFFECT OF TEMPERATURE (°C) ON SPORE PRODUCTION
### (Sporulation Temperatures: 25, 30, 37, 42, 45, 50)

### SPORE PRODUCTION RATING

| Strain | None* Visible | Light | Mod-erate | Heavy | Mod-erate | Light | None* Visible |
|---|---|---|---|---|---|---|---|
| ATCC | | | | | | | |
| 16453 | 25 | 30 | 37 | 42 | | | 45,50 |
| NRRL | | | | | | | |
| 15219 | | 25,30 | 37 | 42 | 45,50 | | |
| 15220 | | 25,30 | 37 | 42 | | | 45,50 |
| 15221 | 30 | | 37 | 42 | | 45 | 50 |
| 15222 | | 30 | | 37,42 | 45,50 | | |
| 15223 | 30 | | 37 | 42 | | 45 | 50 |
| 15224 | 30,37 | | 42 | | | | 45,50 |
| 15225 | 30 | | 37 | 42,45,50 | | | |

*The term "none visible" is used to mean there was growth but without pigmentation; where the temperature is not stated, neither growth nor spores was observed.

Except as noted in the descriptions above, all of the mutants resembled the original strain ATCC 16453. Fully developed spores were globose or subglobose. As the growth of the mycelia lawn increased in extent, isolated patches of hypha became more difficult to distinguish and the occurrence of mature individual spores was more frequent. Concurrently, the number of hyphal strands with developing spores and chains of spores declined. Also, with increased growth, the detection of intercalary spores was more difficult.

Standard Culture Procedure

In order to compare the ability of various strains to produce RSH enzyme preparation, a Standard Culture Procedure is defined as follows. When cultured according to the Standard Culture Procedure, the broths of different fungal varieties may be assayed and directly compared. A fungus strain is cultivated on sterile liquid substrate with the composition:

| | |
|---|---|
| Yeast extract | 0.4% |
| $K_2HPO_4$ | 0.1% |
| $MgSO_4.7H_2O$ | 0.05% |
| Thinned starch* | 20.0% |
| Water | Balance |

*Thinned starch is prepared by gelatinizing corn starch at about 10% (w/v) concentration at pH 6.4-6.6 in the presence of about 2% by volume of 2M sodium acetate buffer containing 3% calcium propionate, cooling to 70°C, adding a small amount of Thermamyl brand alpha amylase and maintaining the 70°C temperature for 1 hour.

Sterile sporulation medium (100 ml, pH 7) is inoculated with organism in 500-ml baffled shake flasks, and the flasks are kept at 42°C for 5-6 days to develop the spore population. The spore flasks are refrigerated until used.

Fermenter inoculum is prepared in 500-ml shake flasks by mixing 100 ml of sterile inoculum medium of the same composition with a volume of spore medium equivalent to about 30 million spores. The seeded inoculum medium is shaken for 16 hours at 42°C, and the resultant inoculum is added at 5% by volume to 10 liters of sterile growth medium in a 14-liter fermenter having an air source and an agitator.

A liquid fermenter medium has the following composition:

| Corn steep liquor | 2.0% |
|---|---|
| $K_2HPO_4$ | 0.15% |
| $MgSO_4.7H_2O$ | 0.05% |
| Pasted corn flour | 0.10% |
| Water | Balance |

After sterilization, one gram each of penicillin G and oxytetracycline is added to the fermenter. When the inoculum is added, 100 grams of sterile granular corn flour is also mixed into the medium.

The fermenter is maintained at 42°C, initially with its agitator at 500 rpm and air flowing through the growth medium at 0.5 volume/volume/minute (vvm). After 18 hours, 200 grams of sterile corn flour and 200 ml of sterile corn steep liquor are added to the fermenter. Air flow and agitator speed are increased to about 1.8 vvm and 60 rpm, respectively. At the 26th hour, 100 grams of sterile corn flour is added to the fermenter. Fermentation is terminated after 73 hours.

The enzyme broth from the fermenter is filtered to remove mycelia and other solid materials. The Standard Culture Procedure is modified for mesophilic fungi which do not grow at 42°C, such fungi being fermented at 37°C.

Determination of Enzyme Activity

Two different assay procedures have been used to measure enzyme activity.

For purposes of this invention, the first test was identified as Standard Assay Procedure Number 1. This assay is the Raw Starch Hydrolysis test which is used to determine the relative activity of the enzyme preparation on granular starch. This test is based on the measurement of reducing sugar (glucose) by the DNSA test (Dinitrosalicyclic acid). In Standard Assay Procedure Number 1, 0.1 milliliter of culture broth (or enzyme solution) is added to one ml of 1% granular corn starch suspended in 0.025 M phosphate buffer, 0.01 M in calcium ion (pH 6.5). The suspension is kept at 50°C for one hour, and then one ml of "DNSA" solution is added (10 grams dinitrosalicyclic acid, 150 ml of 2.66 N sodium hydroxide and 300 grams of potassium sodium tartrate made to 1000 ml with water). The starch suspension is held in boiling water for 5 minutes, and 10 ml of water is added. The optical density at 540 nm is read with a spectrophotometer, e.g., a Spectronic 70 brand colorimeter, as a measure of relative activity. One raw starch hydrolysis (RSH) unit is defined as the amount of reducing sugar required to increase the optical density by 0.1 under the conditions defined. A blank is run to determine dextrose at zero time. Reducing sugar content can be determined from a standard curve of optical density against reducing sugar solutions (0 to 0.3% solution). From this data, an international unit of enzyme activity can be determined. A unit of activity is the amount of enzyme which produces 1 micromole of dextrose per minute under the assay conditions.

Another method is used to avoid effects caused by the granular starch substrate (changes in rate with concentration, difficulties with mixing and clarifying the hydrolyzate, etc.). For purposes of this invention, this method is identified as Standard Assay Procedure Number 2. This is also referred to as the 10 D.E. maltodextrin test. This is a screening test that uses 10 D.E. maltodextrin as a substrate rather than granular starch. One-tenth milliliter of enzyme preparation, diluted if necessary, containing 0.06 to 1.1 units is added to 0.9 ml of substrate solution preheated at 50°C for 5 minutes. The substrate solution consists of 40 parts by volume 0.25M sodium acetate buffer (ph 5.5) and 50 parts by volume of 4% by weight 10 D.E. maltodextrin in water. The substrate solution is kept at 50°C for 5 minutes before the enzyme solution is added. After 10 minutes, the reaction is quenched by pouring into a pre-heated 16-mm tube and heating in a 100°C water bath for 6 minutes. Glucose is determined by any convenient method, such as glucose reagent kit 15-UV from Sigma Chemical or with an instrument such as the Technicon Autoanalyzer. A blank is run to determine glucose at zero time. The result is corrected for any dilution of the sample. The units are referred to as 10 D.E. units or 10 D.E. maltodextrin units.

RSH Activity of Some Improved Mutants

Using the Standard Culture Procedure and Standard Assay Procedure Number 1 described above, certain of the mutants were found to produce higher titers of RSH enzymatic activity than did the wild type, ATCC 16453 tabulated below:

|  | Units/ml |
| --- | --- |
| ATCC 16453 | 80 |
| NRRL 15219 | 286 |
| NRRL 15220 | 172 |
| NRRL 15222 | 152 |

It can be seen that these mutants exhibit RSH activity from about 2 to about 3½ times that of the wild type.

The following examples are presented to illustrate the methods and compositions of the present invention with the understanding that the invention is not limited to the details disclosed. Throughout the specification and claims, percentages, parts and ratios are by weight and temperatures are in degrees Celsius unless otherwise indicated.

EXAMPLE 1

The mutant strain of H. grisea var. thermoidea, NRRL 15219, was cultivated on sterile liquid substrate with the composition:

| Yeast extract | 0.4% |
| --- | --- |
| $K_2HPO_4$ | 0.1% |
| $MgSO_4 \cdot 7H_2O$ | 0.05% |
| Thinned Starch* | 20.0% |
| Water | Balance |

*Thinned starch was prepared by gelatinizing starch at about 10% (w/v) concentration at pH 6.4-6.6 in the presence of about 2% by volume of 2M sodium acetate buffer containing 3% calcium propionate, cooling to 70°C, adding a small amount of Thermamyl brand alpha amylase and maintaining the 70°C temperature for 1 hour.

Sterile sporulation medium (100 ml, pH 7) was inoculated with organism in 500-ml baffled shake flasks, and the flasks were kept at 42°C for 5-6 days to develop the spore population. The spore flasks were refrigerated until used.

Fermenter inoculum was prepared in 500-ml shake flasks by mixing 100 ml of sterile inoculum medium of the same composition with a volume of spore medium equivalent to about 30 million spores. The seeded inoculum medium was shaken for 16 hours at 42°C, and the resultant inoculum was added at 5% by volume to 10 liters of sterile growth medium in a 14-liter fermenter having an air source and an agitator. The enzyme product of this example was prepared in two fermenters.

The liquid fermenter medium had the following composition:

| Corn steep liquor | 2.0% |
| --- | --- |
| $K_2HPO_4$ | 0.15% |
| $MgSO_4 \cdot 7H_2O$ | 0.05% |
| Pasted corn flour | 0.10% |
| Water | Balance |

After sterilization, one gram each of penicillin G and oxytetracycline was added to each fermenter. When the inoculum was added, 100 grams of sterile granular corn flour was also mixed into the medium.

14

Each fermenter was maintained at 42°C, initially with its agitator at 500 rpm and air flowing through the growth medium at 0.5 volume/volume/minute (vvm). After 18 hours, 200 grams of sterile corn flour and 200 ml of sterile corn steep liquor were added to each fermenter. Air flow and agitator speed were increased to about 1.6-2.2 vvm and 600 rpm, respectively. At the 26th hour, 100 grams of sterile corn flour was added to one of the fermenters. Fermentation was terminated after 73 hours.

The enzyme broth from each fermenter was filtered to remove mycelia and other solid materials. The filtered broth from the two fermenters was pooled and frozen for storage. Thawed enzyme solution was centrifuged to remove any solid that formed. The activity of the filtered broth was 183 units per milliliter, measured by the method described above using 10 D.E. maltodextrin (Standard Assay Procedure Number 2).

The enzyme was used at the rate of 50 10 D.E. units per gram of starch to hydrolyze granular corn starch in a slurry at 26% dry substance. The slurry contained 100 ppm of calcium ion with 0.02% propyl paraben and 0.1% methyl paraben added as preservatives (ppm based on slurry weight). The pH and temperature were maintained at 6.0 and 55°C, respectively, for 96 hours with constant stirring. The results are displayed in Table 2.

TABLE 2

| Reaction Time (hours) | Starch Solubilized (%) | Syrup Dextrose Content (% dry substance basis - dsb) |
|---|---|---|
| 24 | 76 | 94.1 |
| 48 | 86 | 93.7 |
| 72 | 90 | 92.3 |
| 96 | 94 | 88.8 |

EXAMPLE 2

H. grisea var. thermoidea, ATCC 16543 was streaked on agar (pH 7) with the composition:

| Yeast extract | 0.2% |
|---|---|
| Beef extract | 0.2% |
| Malt extract | 0.3% |
| Bacto agar Difco | 1.5% |
| Lintner starch | 1.0% |
| Water | Balance |

Covered plates were inverted and kept at 42°C for 5-6 days; they were refrigerated for storage until needed.

Fermenter inoculum was prepared by adding spores to inoculum medium at the rate of about one-quarter plate per 500-ml flask containing 100 ml of medium, and mixing in a blender. The inoculum medium had the following composition:

| Cottonseed protein isolate Proflo[TM*] | 3.0% |
|---|---|
| Corn flour | 3.0% |

*Traders Protein Division of Traders Oil Mill Company

Two sets of flasks were prepared, one sterilized after the corn flour was added (to gelatinize the corn flour starch) and the other sterilized before the addition of sterile corn flour having its starch granules intact. The flasks were shaken at 42°C for 16 hours, and the inoculum was stored under refrigeration.

Five percent of each inoculum, by volume, was added to 10 liters of sterile growth medium in a 14-liter fermenter having an air source and an agitator. The fermenter medium contained only 4% Proflo brand cottonseed protein isolate in water. At the time of addition of the inoculum, 100 grams of sterile granular corn flour was added, and this was repeated at the end of the 18th and 24th hours. Each fermenter was

EP 0 171 218 B1

maintained at 42°C initially with the agitator at 500 rpm and air flow at 0.5 vvm. After 18 hours, air flow and agitator speed were increased to about 1.6-2.2 vvm and 600 rpm, respectively. Fermentation was terminated after 72 hours.

The enzyme broth from the fermenter was handled as in Example 1. Its activity was 29 10 D.E. units per milliliter, measured as in Example 1.

The enzyme was used at the rate of 15 10 DE units per gram to hydrolyze granular corn starch in a slurry at 26% dry substance. The slurry contained 0.02% propyl paraben and 0.1% methyl paraben added as preservatives (% based on slurry weight). The pH and temperature were maintained at 6.0 and 55°C, respectively, for 96 hours with constant stirring. The results are displayed in Table 3.

TABLE 3

| Reaction Time (hours) | Starch Solubilized (%) | Syrup Dextrose Content (% dry substance basis - dsb) |
|---|---|---|
| 24 | 70 | 95.7 |
| 48 | 86 | 94.8 |
| 72 | 91 | 93.7 |
| 96 | 95 | 93.1 |

EXAMPLE 3

This example illustrates the effect of potentiating fraction on the activity of the glucoamylase of this invention in the hydrolysis of granular starch and thinned starch (10 D.E. maltodextrin). One percent granular starch suspensions were subjected to hydrolysis at 50°C and pH 5.5 with 2 10 D.E. units of glucoamylase fractionated as described above from the enzyme preparation of this invention. A solution of potentiating fraction was added to the suspensions of starch in the amounts shown in Table 4. The dextrose content was measured over a period of four hours. The four-hour results (Table 4) demonstrate clearly the effect of the potentiating enzyme on the glucoamylase in dissolving granular starch and converting it to dextrose.

TABLE 4

| | POTENTIATING FRACTION ADDED (microliters) | GLUCOSE (mg/ml) |
|---|---|---|
| Unfractionated enzyme | ..... | 5.7 |
| Glucoamylase fraction | nil | 1.4 |
| Mixed fractions | 21 | 2.8 |
| | 42 | 4.0 |
| | 84 | 4.7 |
| | 168 | 4.9 |
| | 336 | 6.3 |

A similar test was carried out using 2.5 to 2.8 10 D.E. units of glucoamylase in a 17% (w/v) granular starch suspension under essentially the same reaction conditions for six hours. The three samples tested were unfractionated enzyme of this invention, isolated glucoamylase fraction, and isolated glucoamylase fraction to which potentiating factor (13 micrograms of protein) had been added. The dextrose concentrations of the liquid were 72, 16 and 76 mg/ml, respectively.

For comparison, samples of known commercial glucoamylase preparations were compared using the optimum pH 4.4 and optimum temperatures for each, as recommended by the suppliers. These were Aspergillus niger from Boehringer Mannheim, and Aspergillus oryzae and Rhizopus niveus from Sigma Chemical. The first two were tested at 60°C and the last at 50°C using 1% granular starch slurries, as above, for a period of four hours. The three were compared at 2.4 units, 1.7 units and 2.0 units, respectively, against 2.4 units of purified but unfractionated enzyme of this invention. The glucose content of the compositions were 0.7, 1.7, 1.2 and 5.0 mg/ml, respectively, showing the effectiveness of the enzyme

16

of the present invention on granular starch. In contrast, when the same test was carried out on soluble 10 D.E. maltodextrin, all four enzyme preparations reached 95% dextrose in 1.5 hours, essentially the same endpoint at the same time.

## EXAMPLE 4

This example illustrates the use of glucoamylase preparation of this invention in the hydrolysis of B-grade wheat starch. This starch is obtained as a result of fractionation of wheat starch by centrifugation into two fractions following the removal of gluten. The B-grade starch is cream-colored, resistant to attack by alpha amylase and difficult to handle in processes which call for a high-temperature treatment of starch , as in a "jet" cooker (steam injection heater).

An aqueous slurry of granular B-grade wheat starch at 26% solids and pH 5.5 was stirred at 55°C for 24 hours in the presence of 15 10 D.E. units of enzyme preparation per gram of starch. The syrup separated from the residual solids contained 95.7% dextrose, and 64% of the starch had been dissolved. After 96 hours, the dextrose content was 95.3%, and about 73.0% of the starch was dissolved. At 30 10 D.E. units per gram of starch, the corresponding results were 95.7% dextrose with 71% dissolution of the starch at 24 hours and 94.4% dextrose and 78% dissolution at 48 hours.

With the same conditions at 34.3% starch solids, 15 10 D.E. units per gram gave 96% dextrose in the syrup with 54% dissolution at 24 hours and 95.2% dextrose with 57% dissolution in 48 hours when the conversion was terminated. With 20 10 D.E. units per gram, dextrose contents of 95.1 and 94.2% were attained with dissolution of 55 and 58% at the end of 24 and 48 hours, respectively.

## EXAMPLE 5

This illustrates an important and unobvious difference between the enzyme of this invention and prior procedure in which a glucoamylase preparation was used with an alpha amylase preparation to convert granular starch to glucose. The difference appears in the saccharide distribution of the products formed in the hydrolysis. The data also demonstrate the large improvement obtained in using the enzyme preparation of this invention.

An enzyme preparation of this invention (obtained from ATCC 16453, NRRL 15219) was added at 15 10 D.E. units per gram of starch to a 26% dry substance aqueous slurry of granular corn starch containing 50 ppm calcium ion. Samples were taken at 24, 48 and 96 hours. The data is displayed in Figure 1 (dashed lines). The analysis of the liquid (dry substance basis) is given in Table 5. The starch solids concentration decreased from 26% to about 9%. When the same experiment was repeated with 22.5 10 D.E. units of enzyme per gram, the starch residue was reduced to 6%.

At 16% solids, the dextrose content was over 97% at the end of 24 hours, and the starch residue was less than 1%; in many trials it has been found to be undetectable.

A comparable experiment was carried out using together 0.275% of Novo Thermamyl 60L brand bacterial alpha amylase and 0.275% of Miles Laboratories Diazyme L-100 brand glucoamylase preparation, both based on starch dry weight in corn starch slurry at 27.5% dry substance. A pH of 5.5 and temperature of 60°C were maintained for 120 hours with samples taken at 24 hour intervals. These results are also displayed in Figure 1 (solid line) and in Table 5. The alpha amylase is produced from Bacillus licheniformis and has an optimum pH of 5.5 at 60°C. The glucoamylase is from a strain of Aspergillus identified in the literature as of the A. niger group (Smith et al., Staerke 28 (1976) pp. 243-249). The pH of 5.5 was deemed to be optimum under these conditions for dissolution of the starch. It was observed that glucose content was higher in the liquid phase at lower pH, but less starch was dissolved. As Figure 1 shows, there is a gradual increase in dextrose content (dry substance based on soluble saccharide) in contrast to the present invention in which the dextrose content declined with time. The alpha amylase-glucoamylase combination left 16% of the starch at the end of 72 hours and 14% of the starch after 120 hours.

TABLE 5

| | HOURS | DEXTROSE | D.P.2 | D.P.3 | D.P.4 and above |
|---|---|---|---|---|---|
| Enzyme of this invention | 24 | 97.4% | 2.15% | 0.15% | 0.25% |
| | 48 | 96.6 | 3.06 | 0.16 | 0.14 |
| | 72 | 95.6 | 3.86 | 0.25 | 0.15 |
| Alpha amylase/ glucoamylase | 24 | 89.0 | 3.13 | 2.84 | 5.07 |
| | 48 | 91.5 | 2.51 | 2.52 | 3.21 |
| | 72 | 92.7 | 2.25 | 2.33 | 2.83 |
| | 96 | 94.0 | 2.05 | 1.96 | 1.83 |
| | 120 | 94.5 | 2.07 | 1.83 | 1.62 |

The data of Figure 1 and Table 5 clearly demonstrate a fundamental difference in the mode in which the enzyme of this invention operates to hydrolyze and dissolve granular starch: soluble saccharides formed are more than 95% dextrose for the first 24 hours at least, substantially without formation of D.P. 3 and higher glucose polymers. It is believed that the increase in D.P. 2 saccharides is the result of re-polymerization. In contrast, typical glucoamylase preparation, with alpha amylase as co-acting enzyme to solubilize the granular starch, does not reach 90% dextrose in 24 hours and gradually increases to 94-95% after 96 hours. The relatively high production of D.P. 3 and higher saccharides with leveling off at a substantially higher level than in the present invention is also evident.

Several advantages of the present invention can now be more fully appreciated. Using the enzyme preparation produced by the selected fungi, raw rather than pasted starch is the substrate for glucose formation. By eliminating the need to paste the starch, a very substantial energy reduction is realized. The enzyme preparation is useful for hydrolyzing slurries of raw starch with high weight percentage starch, producing a relatively concentrated glucose solution which requires less energy for water removal than does a more dilute solution. From the start of the hydrolysis, the product in solution is substantially entirely glucose. Especially important is the fact that the enzyme preparation produces very low concentrations of limit dextrins and trisaccharides. These substances do not add to the sweetness of the glucose or derived product, such as high fructose syrup, and may even detract from the sweetness. Thus, the enzyme preparation is ideal from almost every standpoint for producing glucose from starch. The mutant species that have been developed which produce high titers of the enzyme mixture improve the feasibility of using the enzyme preparation for largescale production of glucose.

While the invention has been described in terms of certain preferred embodiments, modifications obvious to one with ordinary skill in the art may be made without departing from the scope of the present invention. For example, it is expected that improved mutants will be continuously developed which produce very high titers of enzyme. It is also likely that as more becomes known about the nature of the enzymes that comprise the RSH enzyme mixture, the genes that produce the enzymes will be cloned and appropriately inserted by recombinant DNA technology into other organisms. For purposes of the invention, improved mutated or recombinant organisms producing the same enzyme mixture are considered to be equivalents of the organisms described herein.

FURTHER FEATURES OF THE INVENTION

In the process for producing a granular starch hydrolysis enzyme preparation from a fungal organism, the carbohydrate substrate may include raw starch to induce the fungal organisms to secrete higher titres of the enzyme preparation, and the medium may contain a nutrient source that provides vitamins and proteins, preferably corn steep liquor. The medium may contain a nitrogen source and inorganic salts conducive to growth of the fungal organism.

In the process fro producing glucose from granular starch, the enzyme preparation is preferably supplied to the granular starch in water to supply between about 25 and about 300, more preferably about 50 to about 100. raw starch hydrolyzing units per gram of starch. The slurry preferably contains between about 15 and about 60 percent by weight granular starch.

Although the highly preferred fungal organism is that designated NRRL 15219, the NRRL 15220 and NRRL 15222 strains are also preferred organisms, and generally these organisms which grow at 37°C or above are advantageous.

18

The glucoamylase enzyme fraction of the invention is preferably the carboxymethyl cellulose-absorbed fraction of the enzyme preparation of the invention. The potentiating enzyme fraction is preferably the fraction of the enzyme preparation of the invention which is not absorbed by carboxymethyl cellulose.

In the process of the invention for producing glucose from a thinned starch hydrolyzate, the thinned starch hydrolyzate liquor preferably has a D.E. of 1 to 25, more preferably 5 to 15, a pH of 5 to 7, a solids content of about 15 to 60% starch on a dry substance basis, the hydrolysis of the starch hydrolyzate being above a temperature of about 55ºC.

## Claims

1. An enzyme preparation obtainable from a substantially pure culture of a fungal organism which is a strain of Humicola grisea var. thermoidae, which enzyme preparation catalyzes the hydrolysis of granular starch substantially directly to glucose, said enzyme preparation being characterized in that

(a) it catalyzes the hydrolysis of granular starch suspended in water at a concentration of 15% by weight starch solids substantially completely to soluble glucose syrup solids containing at least 97% by weight glucose, dry substance basis, when the hydrolysis is carried out at a pH of 5.0 to 7.0 and a temperature of 55°C and without added alpha amylase or added debranching enzyme of the pullulanase, isoamylase or beta amylase type,

(b) it is separable by carboxymethyl cellulose into a first non-adsorbing fraction and a second adsorbing proteinaceous fraction, said first non-adsorbing fraction containing a glucoamylase-potentiating activity and said second adsorbing fraction having a glucoamylase enzyme activity (EC 3.2.1.3) that has an isoelectric point of 8.0 or higher.

2. An enzyme preparation according to Claim 1, characterized in that the strain is any one of the organisms deposited under culture collection of references ATCC 16453, NRRL 15219, NRRL 15220, NRRL 15221, NRRL 15222, NRRL 15223, NRRL 15224 and NRRL 15225, or is a genetically altered strain artificially derived from any of said organisms and having the ability to produce an enzyme preparation as characterized in Claim 1

3. A process for producing a granular starch hydrolysis enzyme preparation from a fungal organism comprising:

(i) germinating spores of a fungal organism which is a strain of Humicola grisea var. thermoidae which secretes an enzyme preparation as claimed in Claim 1,

(ii) fermenting a carbohydrate substrate with said germinated fungal organisms in a nutrient medium to produce a broth containing said secreted enzyme, and

(iii) separating said enzyme preparation from said fermenting fungal organisms,

thereby obtaining the granular starch hydrolysis enzyme preparation.

4. A process according to Claim 3 wherein the fungal organism is a strain deposited under reference ATCC 16453, NRRL 15219, NRRL 15220, NRRL 15221, NRRL 15222, NRRL 15223, NRRL 15224 and NRRL 15225, or is a genetically altered strain artificially derived from any of said organisms and having the ability to produce an enzyme preparation as characterized in Claim 1.

5. A process for producing glucose from granular starch characterized by adding an enzyme preparation as claimed in Claim 1 or 2 to a slurry of granular starch in water to hydrolyze the same to glucose.

6. A fungal organism which is a strain of Humicola grisea var. thermoidae deposited under reference NRRL 15219, NRRL 15220, NRRL 15221 NRRL 15222, NRRL 15223, NRRL 15224 and NRRL 15225, or is a genetically altered strain artificially derived from any of said organisms and having the ability when cultured by the Standard Culture Procedure as defined herein to produce an enzyme preparation as characterized in Claim 1 having an RSH (Raw Starch Hydrolysis) activity of 120 units/ml or more as assayed by the Standard Assay Procedure Number 1 as defined herein.

7. A glucoamylase enzyme fraction obtainable from a substantially pure culture of a fungal organism which is a strain of Humicola grisea var. thermoidae, characterized as having an isoelectric point of about 8.0 or higher than 8 0, capable of being adsorbed on carboxymethyl cellulose and exhibiting maximum activity at a pH of between 5.0 and 7.0.

EP 0 171 218 B1

**8.** A process for producing glucose from a thinned starch hydrolyzate comprising adding an enzyme fraction as claimed in Claim 7 to a thinned starch hydrolyzate liquor to hydrolyze the same to glucose.

**9.** A potentiating enzyme fraction which along with a glucoamylase enzyme obtainable from a substantially pure culture of a fungal organism which is a strain of Humicola grisea var. thermoidae, catalyzes the hydrolysis of granular starch suspended in water, at a concentration of 15% by weight starch solids, substantially completely to soluble glucose syrup solids containing at least 97% by weight glucose, dry substance basis, when the hydrolysis is carried out at a pH of 5.0 to 7.0 and at a temperature of 55°C and without added alpha-amylase or added debranching enzyme of the pullulanase, isoamylase or beta-amylase type.

**Patentansprüche**

**1.** Ein Enzympräparat erhältlich von einer substantiell reinen Kultur eines Pilzorganismus, welcher ein Stamm von Humicola grisea var. thermoidae ist, wobei das Enzympräparat die Hydrolyse von körniger Stärke im wesentlichen direkt zu Glucose katalysiert, das Enzympräparat gekennzeichnet dadurch, daß
a) es katalysiert die Hydrolyse von körniger Stärke, suspendiert in Wasser bei einer Konzentration von 15 Gewichtsprozent Stärkesubstanz im wesentlichen vollständig zu löslichen Glucosesirupkörpern mit mindestens 97 Gewichtsprozent Glucose, Trockensubstanzbasis, wenn die Hydrolyse betrieben wird bei einem pH-Wert von 5,0 - 7,0 und einer Temperatur von 55°C und ohne zugefügte Alphaamylase oder zugefügtes Nichtverzweigungsenzym der Pullulanase, Isoamylase oder Betamylasetyp,
b) es ist separierbar durch Carboxymethyl-Cellulose in eine erste nicht absorbierende Fraktion und eine zweite absorbierende proteinartige Fraktion, genannte erste nicht absorbierende Fraktion mit einer Glucoamylase verstärkenden Aktivität und genannte zweite absorbierende Fraktion mit einer Glucoamylase Enzymaktivität (EC 3.2.1.3), welche einen isoelektrischen Punkt von 8.0 oder höher besitzt.

**2.** Ein Enzympräparat gemäß Anspruch 1, dadurch gekennzeichnet, daß der Stamm einer der Organismen ist,die unterlegt sind unter der Kultursammlung von Referenzen ATCC 16453, NRRL 15219, NRRL 15220, NRRL 15221, NRRL 15222, NRRL 15223, NRRL 15224 und NRRL 15225, oder es ist ein genetisch veränderter Stamm künstlich erhalten von einem der genannten Organismen und besitzt die Fähigkeit, ein Enzympräparat zu erzeugen, wie es in Anspruch 1 charakterisiert ist.

**3.** Ein Prozeß zur Erzeugung eines Hydrolyseenzympräparats körniger Stärke von einem Pilzorganismus, beinhaltend:
(i) Keimung von Sporen eines Pilzorganismus, welcher ein Stamm von Humicola grisea var. thermoidae ist, welcher ein Enzympräparat abscheidet, wie es in Anspruch 1 beansprucht ist,
(ii) Fermentierung eines Carbohydratsubstrats mit vorgenanntem gekeimten Pilzorganismus in einem Wuchsmedium zur Erzeugung einer Brühe mit genanntem abgeschiedenen Enzym, und
(iii) Trennung des genannten Enzympräparats von genanntem fermentierten Pilzorganismus, wobei das granulare Stärkehydrolyseenzympräparat erhalten wird.

**4.** Ein Verfahren gemäß Anspruch 3, wobei der Pilzorganismus ein Stamm ist, welcher hinterlegt ist unter der Referenz ATCC 16453, NRRL 15219, NRRL 15220, NRRL 15221, NRRL 15222, NRRL 15223, NRRL 15224 und NRRL 15225, oder einem genetisch verändertem Stamm, welcher künstlich erhalten wird von einem der oben genannten Organismen und welcher die Fähigkeit zur Erzeugung eines Enzympräparates, wie es in Anspruch 1 charakterisiert ist, besitzt.

**5.** Ein Prozeß zur Glucoseerzeugung aus körniger Stärke, gekennzeichnet durch das Hinzufügen eines Enzympräparates, wie es in Anspruch 1 oder 2 beansprucht ist, zu einem Gemisch von körniger Stärke in Wasser, um die gleiche Glucose zu hydrolysieren.

**6.** Ein Pilzorganismus vom Stamm von Humicola grisea var. thermoidae, hinterlegt unter der Referenz NRRL 15219, NRRL 15220, NRRL 15221, NRRL 15222, NRRL 15223, NRRL 15224 und NRRL 15225 oder einem genetisch veränderten Stamm, künstlich erhalten von einem der vorgenannten Organismen,und welcher die Fähigkeit besitzt, wenn er mit dem Standard-Kultur-Verfahren, wie es hierin definiert ist, kultiviert wird, ein Enzympräparat zu erzeugen, wie es in Anspruch 1 charakterisiert ist, mit

20

einer RSH-(Rohstärkehydrolyse)Aktivität von 120 Einheiten pro ml oder mehr besitzt, geprüft mit dem Standard-Prüfungs-Verfahren Nr. 1, wie es hierin definiert ist.

7. Eine Glucoamylaseenzymfraktion, erhältlich von einer im wesentlichen reinen Kultur eines Pilzorganismus, welcher ein Stamm von Humicola grisea var. thermoidae ist, gekennzeichnet dadurch, daß er einen isoelektrischen Punkt von ungefähr 8,0 oder höher als 8,0 hat und fähig ist, an Carboxymethyl-Cellulose absorbiert zu werden und eine maximale Aktivität bei einem pH zwischen 5,0 und 7,0 zeigt.

8. Ein Prozeß zur Glucoseerzeugung von einem verdünnten Stärkehydrolysat, beinhaltend die Zugabe einer Enzymfraktion, wie es in Anspruch 7 beansprucht ist, zu einer verdünnten Stärkehydrolysatflüssigkeit, um die gleiche Glucose zu hydrolysieren.

9. Eine verstärkende Enzymfraktion, welche gemeinsam mit einem Glykoamylaseenzym erhältlich ist von einer im wesentlichen reinen Kultur eines Pilzorganismus, welcher ein Stamm von Humicola grisea var. thermoidae ist, katalysiert die Hydrolyse von körniger Stärke, suspendiert in Wasser, bei einer Konzentration von 15 Gewichtsprozent Stärkesubstanz im wesentlichen vollständig zu löslicher Glucosesirupsubstanz mit mindestens 97% Gewichtsprozent Glucose auf Trockensubstanzbasis, wenn die Hydrolyse ausgeführt wird bei einem pH von 5,0 - 7,0 und einer Temperatur von 55°C und ohne hinzugefügte Alpha-Amylase oder zugeführte nicht verzweigende Enzyme von Pullulanase, Isoamylase oder Beta-Amylasetypus.

**Revendications**

1. Préparation enzymatique que l'on peut obtenir à partir d'une culture d'un organisme fongique qui est une souche Humicola grisea var. thermoidae, laquelle préparation enzymatique catalyse l'hydrolyse d'amidon granulaire directement pratiquement en glucose, ladite préparation enzymatique étant caractérisée en ce que
    (a) elle catalyse l'hydrolyse de l'amidon granulaire en suspension dans l'eau à une concentration de 15% en poids de solides d'amidon pratiquement etr complètement en solides de sirop de glucose solubles contenant au moins 97% en poids de glucose, sur la base de substance solide, quand on effectue l'hydrolyse à un pH de 5,0 à 7,0 et une température de 55°C et sans alpha-amylase ajoutée ou enzyme de déramification ajoutée de type pullulanase, isoamylase ou béta-amylase,
    (b) elle est séparable grâce à la carboxyméthylcellulose en une première fraction non adsorbante et une seconde fraction adsorbante protéagineuse, ladite première fraction non adsorbante contenant une activité potentialisant la glucoamylase et ladite seconde fraction adsorbante ayant une activité de glucoamylase (EC 3.2.1.3) qui a un point isoélectrique de 8,0 ou supérieur.

2. Préparation enzymatique selon la revendication 1, caractérisée en ce que la souche est l'un quelconque des organismes déposés sous les références de la collection de culture ATTC 16 453, NRRL 15 219, NRRL 15 220, NRRL 15 221, NRRL 15 222, NRRL 15 223, NRRL 15 224 et NRRL 15 225 ou est une souche modifiée génétiquement dérivée artificiellement de l'un quelconque desdits organismes et ayant la capacité de produire une préparation d'enzyme selon la revendication 1.

3. Procédé pour produire une préparation enzymatique de l'hydrolyse de l'amidon granulé provenant d'un organisme fongique comprenant les opérations consistant:
    (i) à faire germer des spores d'un organisme fongique qui est une souche Humicola grisea var. thermoidae qui secrète une préparation enzymatique selon la revendication 1.,
    (ii) à faire fermenter un substrat de carbohydrate avec lesdits organismes fongiques germés dans un milieu nutritif pour produire un bouillon contenant ladite enzyme sécrétée, et
    (iii) à séparer ladite préparation enzymatique desdits organismes fongiques en fermentation,
    obtenant ainsi la préparation enzymatique de l'hydrolyse de l'amidon cellulaire.

4. Procédé selon la revendication 3, dans lequel l'organisme fongique est une souche déposée sous les références ATTC 16 453, NRRL 15 219, NRRL 15 220, 15 221, NRRL 15 222, NRRL 15 223, NRRL 15 224 et NRRL 15 225, ou est une souche modifiée génétiquement dérivée artificiellement de l'un quelconque desdits organismes et ayant la capacité de produire une préparation enzymatique telle que caractérisée dans la revendication 1.

5. Procédé pour produire du glucose à partir d'amidon granulaire, caractérisé par l'opération consistant à ajouter une préparation enzymatique selon la revendication 1 ou 2 à une suspension d'amidon granulaire dans l'eau pour hydrolyser celui-ci en glucose.

6. Organisme fongique qui est une souche Humicola grisea var. thermoidae déposé sous les références NRRL 15 219, NRRL 15 220, NRRL 15 221, NRRL 15 222, NRRL 15 223, NRRL 15224 et NRRL 15 225, ou est une souche modifiée génétiquement dérivé artificiellement de l'un quelconque desdits organismes et ayant la capacité quand il est cultivé selon le protocole normalisé de culture tel que défini dans l'invention pour produire une préparation enzymatique selon la revendication 1, ayant une activité RSH (hydrolyse de l'amidon brut) de 120 unités/ml ou supérieure, dosée par le protocole de dosage normalisé numéro 1 tel que défini dans l'invention.

7. Fraction enzymatique glucoamylase que l'on peut obtenir à partir d'une culture pratiquement pure d'un organisme fongique qui est une souche Humicola grisea var. thermoidae, caractérisée en ce qu'elle a un point isoélectrique d'environ 8,0 ou supérieur à 8,0, capable d'être adsorbée sur de la carboxyméthylcellulose et présentant une activité maximale à un pH compris entre 5,0 et 7,0.

8. Procédé pour produire du glucose à partir d'un hydrolysat d'amidon raffiné comprenant l'opération consistant à ajouter une fraction enzymatique selon la revendication 7 à une liqueur d'hydrolysat d'amidon raffiné pour hydrolyser celui-ci en glucose.

9. Fraction enzymatique potentialisante qui, conjointement avec une glucoamylase que l'on peut obtenir à partir d'une culture pratiquement pure d'un organisme fongique qui est une souche Humicola grisea var. thermoidae, catalyse l'hydrolyse de l'amidon granulaire en suspension dans l'eau, à une concentration de 15% en poids de solides d'amidon, pratiquement complètement en solides de sirop de glucose solubles contenant au moins 97% en poids de glucose, sur la base de substance sèche, quand on effectue l'hydrolyse à un pH de 5,0 à 7,0 et à une température de 55°C et sans alpha-amylase ajoutée ou d'enzyme de déramification ajoutée du type pullulanase, isoamylase ou béta-amylase.

PERCENT GRANULAR STARCH
DISSOLVED
—·—· ENZYME PREPARATION
OF THIS INVENTION
············ COMBINATION OF
COMMERCIAL ENZYMES

PERCENT DEXTROSE DRY
SUBSTANCE BASED IN SYRUP
———— ENZYME PREPARATION
OF THIS INVENTION
———— COMBINATION OF
COMMERCIAL ENZYMES

REACTION TIME, HRS.